# EUROPÄISCHE PATENTSCHRIFT

(11) **EP 2 288 361 B1**
(45) Veröffentlichungstag und Bekanntmachung des Hinweises auf die Patenterteilung: **21.08.2013**
(21) Anmeldenummer: 09742098.8
(22) Anmeldetag: 06.05.2009
(51) Int. Cl.: A61K 36/185, A61P 31/12, A61P 29/00, A61P 37/08

(54) **ZISTROSENEXTRAKT MIT ANGEREICHERTEN SEKUNDÄREN PFLANZENINHALTSSTOFFEN**
CISTUS EXTRACT CONTAINING ENRICHED SECONDARY PLANT INGREDIENTS
EXTRAIT DE CISTE CONTENANT DES INGRÉDIENTS VÉGÉTAUX SECONDAIRES ENRICHIS

(30) Priorität: 06.05.2008 EP 08155699
(43) Veröffentlichungstag der Anmeldung: 02.03.2011
(73) Patentinhaber: Finzelberg GmbH & Co. KG, 56626 Andernach (DE)
(72) Erfinder: FEISTEL, Björn, 56626 Andernach (DE); WALBROEL, Bernd, 53639 Königswinter (DE)
(74) Vertreter: von Kreisler Selting Werner
(86) Internationale Anmeldenummer: PCT/EP2009/055487
(87) Internationale Veröffentlichungsnummer: WO 2009/135880

(56) Entgegenhaltungen:
- EP-A- 1 837 029
- WO-A-2007/110133
- DE-U1-202006 004 872
- DEMETZOS C ET AL: "Polyphenolic glycosides from Cistus creticus L. leaves" ANNALES PHARMACEUTIQUES FRANCAISES 1989 FR, Bd. 47, Nr. 5, 1989, Seiten 314-318, XP009119988 ISSN: 0003-4509
- KUPELI ET AL: "Flavonoids with anti-inflammatory and antinociceptive activity from Cistus laurifolius L. leaves through bioassay-guided procedures" JOURNAL OF ETHNOPHARMACOLOGY, ELSEVIER SCIENTIFIC PUBLISHERS LTD, IE, Bd. 112, Nr. 3, 4. Juli 2007 (2007-07-04), Seiten 524-530, XP022142068 ISSN: 0378-8741
- YAMAGUCHI MASAYOSHI ET AL: "Anabolic effects of bee pollen Cistus ladaniferus extract in osteoblastic MC3T3-E1 cells in vitro" JOURNAL OF HEALTH SCIENCE - EISEI KAGAKU, PHARMACEUTICAL SOCIETY OF JAPAN, JP, Bd. 53, Nr. 5, 1. Oktober 2007 (2007-10-01), Seiten 625-629, XP009111616 ISSN: 1344-9702
- BLANK I: "EXTRACT AUS CISTROSEN LINDERT HALSSCHMERZEN//CISTUS INCANUS EXTRACT RELIVES SORE THROAT" DEUTSCHE APOTHEKER ZEITUNG, DEUTSCHER APOTHEKER VERLAG, STUTTGART, DE, Bd. 145, Nr. 46, 17. November 2005 (2005-11-17), Seite 40/41, XP001247104 ISSN: 0011-9857
- HOC S: "Extract from Cistus incanus against influenza pandemic?" ZEITSCHRIFT FUER PHYTOTHERAPIE, HIPPOKRATES VERLAG IN MVS MEDIZINVERLAGE, DE, Bd. 28, Nr. 3, 1. Januar 2007 (2007-01-01), Seiten 142-143, XP009109153 ISSN: 0722-348X
- RICHTER TH: "Rock Rose (Cistus) cotra dysentery. Ancient knowledge revisited" MMW FORTSCHRITT DER MEDIZIN, URBAN UND VOGEL MEDIEN UND MEDIZIN VERLAGSGESELLSCHAFT, DE, Bd. 142, Nr. 47, 23. November 2000 (2000-11-23), Seiten 47-48, XP009111617 ISSN: 1438-3276
- SARACINI E ET AL: "Simultaneous LC-DAD and LC-MS Determination of Ellagitannins, Flavonoid Glycosides, and Acyl-Glycosyl Flavonoids in Cistus salvifolius L. Leaves" CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, Bd. 62, Nr. 5-6, 1. September 2005 (2005-09-01), Seiten 245-249, XP019358506 ISSN: 1612-1112

## Beschreibung

Die vorliegende Erfindung betrifft angereicherte Extrakte aus Cistus cresticus L. und daraus hergestellte Zubereitungen, Verfahren zu ihrer Herstellung und ihre Verwendung.

In der Familie der Cistaceae bilden die Zistrosen (Cistus) eine Gattung mit 18 bis 20 Arten. Die Untergattung *Cistus creticus L. ssp. eriocephalus* wird als aromatisch riechender immergrüner Strauch bis 1m groß. Seine wechselständigen, eiförmig-lanzettlichen Blätter und radiärsymetrischen, fünfzähligen Blüten sind üblich für die ganze Familie, die rosarote oft pinkfarbene bis hellviolette Färbung der Blüten ist jedoch bezeichnend für diese Untergattung. Bevorzugter Lebensraum ist der östliche Mittelmeerraum.

Die Volksmedizin nutzte die Wirkung dieser Pflanze in unterschiedlichster Weise, hauptsächlich jedoch als Adjuvans bei topischem, oft allergiebedingtem Juckreiz, sowie zur Prävention und Wundbehandlung bei bakteriellen Infektionen und Mykosen. Traditionell werden hierfür wäßrige Abkochungen (Dekokte) der frühjährlichen oberirdischen Pflanzenteile verwendet. In jüngeren Veröffentlichungen wurde über den erfolgreichen Einsatz von Cistus-Lösungen zur Behandlung von Neurodermitis, Akne vulgaris, sowie bei entzündlichen Erkrankungen des Mund- und Rachenraumes berichtet. Bekannt ist das Cistus-Kraut durch seine Verwendung als Tee (z.B. Cystus® Bio Teekraut). Hier werden ca. 2,5 g Cistus incanus-Kraut auf 1 Liter Heißwasser als Dosierung empfohlen. Eigene Analysen ergaben, dass hiermit ca. 1 g Extraktivstoffe in gelöster Form mit ca. 16% Polyphenolen zu sich genommen werden. Andere Anbieter von Cistus-Kraut, die keine Angabe zur Species vornehmen, beziehen ihre Ware aus Wildsammlung und liefern entsprechend schwankende Extraktivstoffe von 0,7-1,5 g bzw. schwankende Polyphenolgehalte von 11 - 21%. Diese nicht standardisierten Mischungen repräsentieren den natürlichen Polymorphismus der Pflanzen und bieten keine Voraussetzung zur Herstellung eines standardisierten Pflanzenextraktes.

Eine nähere Spezifizierung in die Varietäten, respektive Subspezies, wurde erst in jüngerer Zeit dringend notwendig und durch eine sortenschutzrechtliche Abgrenzung der Sorte *Cistus incanus L. ssp. Pandalis*^{®} bzw. unter der Marke Cystus^{®} relevant, welche insbesondere für den Laien von Cistus creticus L. ssp. eriocephalus (auch bekannt unter *Cistus incanus L. ssp. tauricus*) nicht zu unterscheiden ist.

Die historisch ältesten Überlieferungen für Cistus-Präparationen befassen sich mit Labdanum, einem Harz, welches überwiegend aus den Blättern und Zweigen von Cistus ladaniferus L., Cistus monspliensis L. und Cistus incanus L. gewonnen wurde. Es wurde auf Grund seines angenehmen Aromas gerne verräuchert, fand jedoch auch medizinische Anwendung als Expectorans bei Atemwegskatarrhen, sowie in Plastri und Unguenta zur Wundbehandlung.

Phytochemische Untersuchungen der Gattung Cistus konzentrierten sich in der Folge häufig auf die Analyse der lipophilen Bestandteile, insbesondere des ätherischen Öles und der Harzbestandteile. Hierin finden sich übliche Strukturen des Terpenstoffwechsels wie Mono-, Sesqui- und Diterpene, sowie Alkohole und Ester. Weitere Untersuchungen befassten sich mit den Polyphenolfraktionen der Flavonoide und Gerbstoffe. Hierbei wurden Kämpferol, Quercetin und Apigenin als überwiegende Grundstrukturen analysiert. Als sehr polyphenolreiche Species haben sich Muster von Cistus creticus ssp. herausgestellt. Eigene Untersuchung haben überraschenderweise ergeben, dass sich im Polyphenol-Fingerprint von Cistus creticus ssp. eriocephalus ein bekanntes Muster wieder findet: ähnlich wie bei Grüntee-Extrakten finden sich im Bereich der wertgebenden Oligomer-Fraktionen Catechin-Derivate wie Epigallocatechin oder das Epigallocatechingallat (EGCG). Der Gesamtgehalt an Polyphenolen in der Pflanze wird im Allgemeinen mit 4% angegeben, wobei jedoch der Anteil in den Blättern deutlich höher liegt.

Für Polyphenole ist allgemein ihr antioxidatives Verhalten bekannt. Hierunter versteht man das Potential, freie Radikale abzufangen. Im menschlichen Körper sind es besonders "reaktive Sauerstoffspezies", die hervorgerufen durch Umwelteinflüsse (UV-Strahlung, chemische Noxen) oder einseitige, falsche Ernährung in besonders hohem Maße auftreten können. Nach heutiger wissenschaftlicher Kenntnis stehen viele Erkrankungen im Zusammenhang mit "reaktiven Sauerstoffspezien", da die von diesen Radikalen hervorgerufenen Zellschädigungen eine Vielzahl von Krankheiten (z.B. Immunkrankheiten, Mentale Erschöpfung, Arthritis) auslösen können, wenn sie nicht durch "antioxidative Radikalfänger" limitiert werden. Daher liefert die Messung der antioxidativen Kapazitäten häufig richtungsweisende Werte. Hohe Polyphenolgehalte weisen auf ein hohes antioxidatives Potential hin.

Aktuelle Forschungen zeigen für einen Extrakt der Zistrose (CYSTUS^{®}052; hergestellt aus Cistus incanus PANDALIS, 26% Polyphenole, weniger als 2% Monomere (Gallsäure, Epigallocatechin, Catechin und Epicatechin)), dass dieser die Vermehrung von Viren der Virusgrippe (Echte Grippe, Influenza) *in-vitro* signifikant hemmt, ohne dass Resistenzen auftreten (Ehrhardt C. et al., Antiviral Res., 76, 38-47, 2007). Die Überprüfung dieser Ergebnisse in vivo (Maus-Modell) konnte ebenfalls belegt werden (Droebner, K. et al., Antiviral Res. 76, 1-10, 2007).

Handelsprodukte wie Cistus Incanus Kapseln (Fa. LR Health & Beauty Systems) mit einfachen wäßrigen Extrakten (20-25% Polyphenole; Droge-Extrakt-Verhältnisse von 2 - 4:1) repräsentieren den aktuellen Stand der Technik. Eigene Untersuchungen bestätigten, das wässrige Extraktionen von Cistus incanus ssp. tauricus oder Cistus creticus eriocephalus in Abhängigkeit der verwendeten Krautqualität Extrakte mit 20-28% Polyphenolen liefern können.

Um Wirkungen von Cistuskraut-Zubereitungen vergleichend zu bewerten, ist daher eine eindeutige Zuordnung ihrer Species notwendig. Ferner gehören Angaben zur Herstellung (Auszugsmittel; Droge-Extrakt-Verhältnis) sowie eine analytische Charakterisierung (Polyphenole) als Voraussetzung für ein standardisiertes Produkt. Nur eine reproduzierbare, standardisierte Extraktqualität sichert bei arzneilichen Anwendungen ein gleichbleibendes Therapieschema. Aufgabe der Erfindung war die Bereitstellung eines Verfahrens zu Herstellung eines Zistrosenkraut-Extraktes mit hohem Gehalt, das in reproduzierbarer Weise standardisierte Extrakte bevorzugt mit mindestens 40% (m/m) Polyphenolen ergibt.

Gelöst wird die Aufgabe durch ein Verfahren zur Herstellung eines Extraktes aus *Cistus creticus L.* mit folgenden Schritten:
- Extrahieren von Pflanzenteilen von *Cistus creticus L.* mit einem Extraktionsmittel ausgewählt aus der Gruppe Wasser, Methanol, Ethanol, 1-Propanol, 2-Propanol und Mischungen davon, wobei der Gehalt an Alkohol nicht mehr als 50% (V/V) beträgt
- Entfernen von Extraktionsrückständen
- Zumindest teilweises Entfernen des Extraktionsmittels
- Rücklösen in einem wässrigen Lösungsmittel mit mindestens 50% (m/m) Wasser und Entfernen von unlöslichen Bestandteilen
- selektives Anreichern durch mindestens einen der folgenden Schritte
   a) eine Zwei-Phasen-Extraktion, wobei
      - bei einer Festphasenextraktion die Polyphenole an die Festphase binden und durch lipophilere Lösemittel eluiert werden und
      - bei einer Flüssig-Flüssig-Extraktion die organische Phase die Produktphase bildet,
   b) eine Membranfiltration, wobei das Retentat die Produktphase bildet. Wünschenswert für einen solchen Herstellprozess ist eine nahezu gleichbleibende Qualität des pflanzlichen Einsatzmaterials. Diese wird erreicht durch Analyse der Polyphenol-Gehalte (bestimmt mittels Folin-Reagenz und berechnet als Gallussäure analog der Methodik des Europäischen Arzneibuchs, Kapitel PH. EUR. (2.8.14)) und mischen von Chargen.

Bevorzugt wird die Droge einer Mahlung und Windsichtung unterzogen, um eine Anreicherung des Blattmaterials gegenüber Stängelanteilen zu erreichen. Die Blätter weisen höhere Gehalte an Polyphenolen auf. Hierdurch wird eine Verbesserung um ca. 30% erreicht. *Cistus creticus* L. ssp *eriocephalus* wird bevorzugt eingesetzt. Bevorzugt werden oberirdische Pflanzenteile verwendet. Generell wird Ausgangsmaterial mit einem Gehalt von min. 12% Polyphenolen bevorzugt.

Geeignete Extraktionsmfttel sind Wasser, Methanol, Ethanol, 1-Propanol, 2-Propanol und Mischungen, davon wobei der Gehalt an Alkohol bei nicht mehr als 50% (V/V), bevorzugt nicht mehr als 40% (V/V) oder nicht mehr als 30% (v/v) liegt.

In einer Ausführungsform wird die Extraktion bei einer erhöhten Temperatur durchgeführt. Temperaturen von 40 bis 80°C sind besonders bevorzugt, um eine besonders hohe Polyphenolausbeute zu erreichen.

Nach der Extraktion wird üblicherweise der Drogenrückstand entfernt; dies kann beispielsweise durch Abfiltrieren oder Absaugen und anschließendes Auspressen des Drogenrückstandes erfolgen. Weitere Möglichkeiten sind dem Fachmann bekannt.

Anschließend wird das Extraktionsmittel zumindest teilweise aus dem erhaltenen Extrakt entfernt. Dies kann beispielsweise durch Abziehen des Lösungsmittels in einem Rotationsverdampfer oder mit Hilfe eines Plattenverdampfers erfolgen. Eine schonende Behandlung ist bevorzugt.

Nach dem Entfernen liegt der Trockensubstanzanteil des verbleibenden Extraktes bevorzugt bei mehr als 50% (m/m).

Im nächsten Schritt erfolgt ein Rücklösen des verbliebenen Rückstandes. Zum Rücklösen sind insbesondere Wasser oder Mischungen von Wasser mit Alkohol besonders geeignet. Das Rücklösemittel enthält zumindest 50% (m/m) Wasser.

Es verbleibt nach dem Rücklösen ein Rückstand, der durch Abfiltrieren, Absaugen, Dekantieren o.ä. abgetrennt wird und verworfen werden kann. Der Rückstand enthält zumindest einen Teil der Tannine (wenig bioaktive Polyphenole).

Anschließend erfolgt ein Anreicherungsschritt. Hierzu kann entweder eine Zwei-Phasenextraktion oder eine Membranfiltration eingesetzt werden.

Bei der Zwei-Phasenextraktion kann es sich um eine Flüssig-Flüssig-Extraktion handeln. Als Extraktionsmittel für die Flüssig-Flüssig-Extraktion hat sich insbesondere n-Butanol bewährt.

Alternativ oder zusätzlich kann auch eine Festphasen-Extraktion vorgenommen werden. Eine Extraktion mit Adsorberharzen ist besonders geeignet. Typische Adsorberharze sind beispielsweise nichtionische hydrophobe Divinylbenzen-Copolymere, aliphatische Esterpolymere und Formophenolpolymere. Solche Adsorber sind kommerziell unter dem Handelsnamen Amberlite^{®} erhältlich. Geeignete Produkte sind die Typen XAD2, XAD4, XAD7HP, XAD16, XAD761 oder XAD1180. Verwendet werden können aber auch analog charakterisierte Harze anderer Hersteller wie von Fa. Diaion (SP-Serie), von Fa. Bayer (Lewatite^{®}) oder von Fa. Miontech (P-Serie) .

Der Harztyp Amberlite^{®} XAD7HP ist dabei besonders bevorzugt.

Alternativ kann eine Membranfiltration durchgeführt werden. Als Membranen eignen sich insbesondere porendefinierte Materialien aus Polyestersulfan, Polypropylen, Polytetrafluorethylen oder Celluloseacetat. Geeignete Porengrößen liegen bei 70 - 100 kD. Diese Membranen können als mehrfach, übereinander angebrachte Filter in Filterplatten eingesetzt werden. Hierbei verbleiben ca. 60% der Polyphenole im Retentat (angereicherte Produktphase). Membranen aus Polyestersulfan sind dabei besonders bevorzugt.

Bei einer Flüssig-Flüssig-Extraktion wird die polyphenolhaltige, wässrige Phase mit einem organischen Lösemittemittel wie n-Butanol behandelt. Dabei gehen die Polyphenol-Substanzen durch intensive Vermischung in die organische Phase über, die organische Phase wird weiter aufgearbeitet.

Bei einer Fest-Phasen-Extraktion verbleiben die Polyphenole selektiv auf der Festphase, bevor diese durch einen Elutionswechsel auf lipophilere Lösemittel wie z.B. Ethanol als flüssige Phase eluiert und abgetrennt werden können, die organische Phase wird weiter aufgearbeitet.

Bei einer Membranfiltration werden die Polyphenole auf Grund ihrer Größe über Poren abgetrennt. Die sekundären Pflanzeninhaltsstoffe werden über ihre Molekülgerüste größenabhängig zurückgehalten. Begleitstoffe wie Salze und einfache Säuren werden mit dem Filtrat abgetrennt. Es verbleibt eine aufkonzentrierte Polyphenol-Fraktion im Retentat.

Anschließend wird der Extrakt von seinem Lösemittel im Vakuum befreit und kann getrocknet werden, um einen Trockenextrakt zu erhalten. Die Trocknung sollte möglichst schonend durchgeführt werden.

Gegenstand der Erfindung ist auch der durch das Verfahren erhältliche Extrakt.

In einer bevorzugten Ausführungsform ist der Extrakt gekennzeichnet durch mindestens eine der folgenden Eigenschaften:
- einem nativen Droge-Extrakt-Verhältnis (DEVnativ) von 4-40:1
- einem ORAC-Wert von größer 3000 µmol Trolox-Equivalent /g
- einem Gesamtpolyphenolgehalt von größer 40%, bevorzugt >60% (m/m) und/oder
- einem Gehalt an Monomeren von mindestens 2% (m/m).

Überraschend wurde gefunden, dass der erfindungsgemäße Cistus-Extrakt seine Wirkung über eine Inhibierung von NF-κB vermittelt.

NF-κB ist ein wichtiger spezifischer Transkriptionsfaktor, hat zahlreiche Zielgene und vermittelt unterschiedlichste Wirkungen. Die Aktivierung von NF-κB gilt als kritisch für die Entstehung von Entzündungen. Aufgrund seiner vielfältigen Funktionen wird NF-κB auch mit zahlreichen Erkrankungen in Zusammenhang gebracht. Dabei ist vielfach unklar, inwieweit die Aktivierung von NF-κB tatsächlich kausal in den Krankheitsprozess eingreift. Bei einigen Arten von Krebserkrankungen wird eine solche Rolle zunehmend als wahrscheinlich angesehen, so dass Bestandteile des NF-κB- Signalweges inzwischen wichtige Zielstrukturen für die Entwicklung neuer Medikamente geworden sind. NF-κB kann an ein spezifisches DNA-Motiv von etwa zehn Basenpaaren, das so genannte κB-Motiv, binden. Die Bindung von NF-κB an das DNA-Motiv führt in den allermeisten Fällen zu einer verstärkten Transkription der davon abhängigen Gene. Man geht derzeit davon aus, dass größenordnungsmäßig etwa 200 verschiedene Gene von NF-κB reguliert werden. Darunter fallen viele Zytokine (z.B. TNF-α und IL-1β) und Adhäsionsmoleküle, die eine bedeutende Rolle bei der Regulation des Immunsystems, insbesondere auch bei Entzündungsreaktionen spielen.

Zu den Stimuli, die eine Aktivierung von NF-κB auslösen können, zählen u.a. virale Antigene (z. B. Lipopolysaccharide). Allergisch-entzündliche Reaktionen verlaufen ebenso über diese NF-κB-Signalkaskade und können durch eine inhibierende Beeinflussung gleichfalls gemindert werden.

Als Testmodell wurde sogenannte A549 Lungenzellen verwendet. Untersucht wurden die Auswirkungen des erfindungsgemäßen Cistus-Extraktes auf die transkriptionelle Aktivität von NF-κ B. Das Zellsystem wurde mit einem Luciferase-Meßsystem gekoppelt. Nach Ermittlung der Blindwerte wurde die synthetische Substanz PMA als Stimulus zugesetzt, welches die Luciferase Genexpression in A549 Zellen deutlich erhöht. Diesem System wurde der Cistus-Extrakt in verschiedenen Dosen zugeführt. Es konnte eine deutlich dosisabhängig Inhibierung festgestellt werden.

| | | | | | |
|---|---|---|---|---|---|
| Konzentration Cistus-Extrakt [µg/mL] | 1 | 10 | 50 | 100 | 150 |
| Inhibierung von NF-κ B [%] | keine | keine | 22 | 42 | 65 |

Da die A549-Zellen Bindungsstellen für zwei mögliche Transkriptionsfaktoren aufweisen, entweder für die NF-κ B oder für AP-1, wurde bezüglich der Spezifität auch die AP-1 Bindungstelle untersucht. Nach Zugabe eines AP-1 Luc Plasmids zur Vermessung der Luciferase-Aktivität wurden folgende Ergebnisse erhalten.

| | | | | | |
|---|---|---|---|---|---|
| Konzentration Cistus-Extrakt [µg/mL] | 1 | 10 | 50 | 100 | 150 |
| Inhibierung von AP-1 [%] | keine | keine | + 52 | + 50 | + 92 (Aktivierung) |

Es wurde eine Aktivierung des AP-1 Transkriptionsfaktors festgestellt. Die Ergebnisse belegen, daß es sich um eine spezifische NF-κ B vermittelte Inhibierung handelt und Meßfehler mit dem System ausgeschlossen sind. Auch die negative Cytotoxizitätsprüfung belegte die nachgewiesenen Ergebnisse.

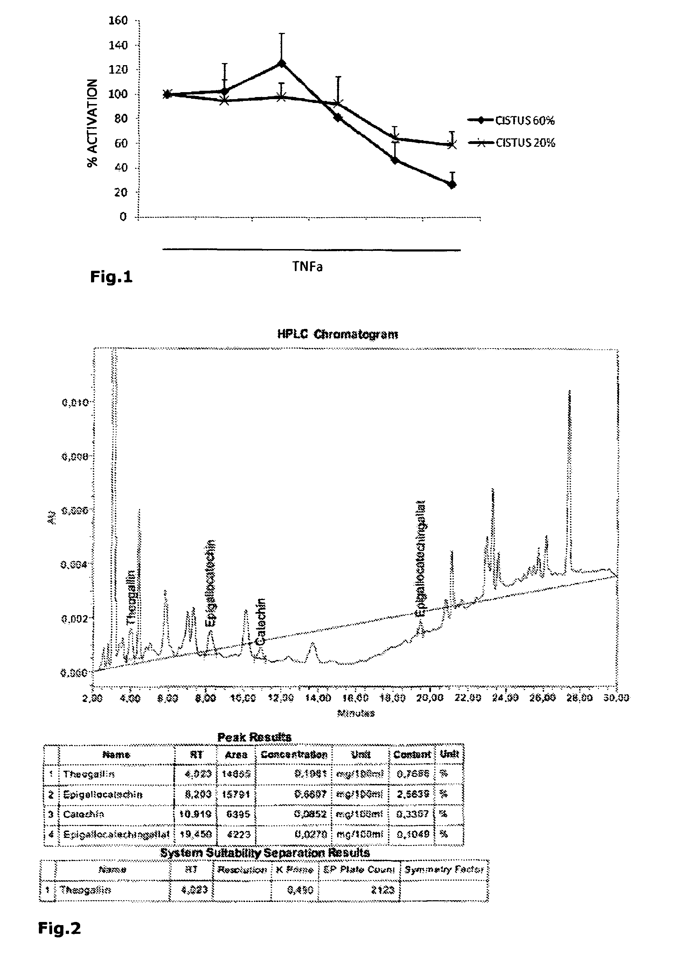

Um den neu entdeckten Wirkmechanismus via NF-κ B zu verifizieren, wurde ein weiteres Modell gewählt, in dem NF-κ B selbst eine Wirkungsvermittlung zu dem aus anti-inflammatorsichen Untersuchungen bekannten Tumornecrosefaktor-alpha (TNF-alpha) vorsieht. Hiernach kann man prüfen, ob Cistus-Extrakt eine NF-κ B induzierte Aktivierung von TNF-alpha beeinflußt (kanonischer Weg). Es wurde eine geklonte 5.1 Zelllinie benutzt, in der sich ein Luciferase-Gen von HIV-1-LTR-Promotor befand. Die Zellen wurden mit steigender Extraktdosierung präinkubiert und für zu 6 Std. mit TNF- Zellen stimuliert. Anschließend wurden die Zellen abgetrennt und Reporter-Aktivität im Luciferase-Modell gemessen. Es konnte eine dosisabhängige Inhibierung der TNFalpha abhängigen HIV-1-LTR-Gen-Transkription ermittelt werden (siehe Figur 1). Dabei erwies sich der erfindungsgemäße Extrakt mit >40% Polyphenolen deutlich potenter als ein wässriger Extrakt mit >20% Polyphenolen (Stand der Technik).

Der Einfluss des erfindungsgemäßen Cistus-Extraktes auf den NF-kappaB Signalweg wurde nachweislich bestätigt.

Durch das Verfahren wird ein Extrakt erhalten, der für folgende Anwendungen besser geeignet ist, als bislang bekannte Extrakte:
- Behandlung oder Prophylaxe von viralen Erkrankungen
- Behandlung oder Prophylaxe von allergisch bedingten Entzündungsreaktionen, insbesondere allergische Rhinitis.
- Behandlung oder Prophylaxe der eine Erkältung begleitenden Krankheitsbilder wie Rhinitis, Sinusitis, Laryngitis, Chorditis
- Behandlung des postviralen Erschöpfungssyndroms
- Behandlung von Influenza, Cholera, Clostridien-Myositis, Enteritis necroticans, Herpes-Infektionen.

Gegenstand der Erfindung ist auch eine Extraktzubereitung, die den erfindungsgemäßen Extrakt enthält.

Gegenstand der Erfindung ist auch eine pharmazeutische Zubereitung oder ein Lebensmittel/Nahrungsergänzungsmittel, das den erfindungsgemäßen Extrakt oder die erfindungsgemäße Extraktzubereitung enthält.

Der erste Kontakt eines Virus mit dem potentiellen Wirt findet üblicherweise über die Schleimhäute statt. Dementsprechend führt eine lokale Applikation auf den Schleimhäuten zu guten Erfolgen. Neben den bisherigen Anwendungen im Mund- und Rachenbereich als Lutschpastille oder als Gurgellösung bei Mandel- und/oder Zahnfleischentzündung, zeigte sich überraschenderweise die Anwendung bei Krankheitsbildern wie Nasenschleimhaut-, Nasennebenhöhlen, Kehlkopf- oder Stimmbandentzündung, die eine Erkältung jedoch häufig begleiten, als wirksam.

Überraschenderweise wurde gefunden, dass erfindungsgemäß hergestellte Cistus-Extrakte eine hohe Hemmwirkung auf die Neuraminidase-Aktivität zeigen.

Neuraminidasen sind eine Familie von Enzymen von Influenza- (Typ A und B) und anderen Viren, die terminale Sialinsäurereste von den Glycoproteinen der Zelloberflächen von Virenwirtszellen und den Viren selber abspalten. Dieser Vorgang befördert die Freisetzung von Tochterviren aus den infizierten Zellen. Sogenannte Neuraminindase-Inhibitoren sind Medikamente, die diesen Prozess der Abspaltung von der Wirtszelle nach einer viralen Infektion verlangsamen und somit die (systemisch) freie Viruslast mindern. Als wichtig gilt dafür generell, dass das Medikament innerhalb kürzester Zeit nach den ersten Symptomen einzunehmen, da zu diesem Zeitpunkt die körpereigene Immunabwehr noch nicht erschöpft ist und durch eine medikamentöse Verminderung der Viruslast effizienter wird. Zu diesem Medikamententyp gehören Tamiflu^{®} (Oseltamivir) und Relenza^{®} (Zanamivir), welchen die Hoffnung gilt, bei einer eventuellen Pandemie durch das Vogelgrippe Virus H5N1 eine erste Eindämmung bewirken zu können. Leider weisen diese chemischen Neuraminidase-Inhibitoren häufig Nebenwirkungen wie Übelkeit, Magenschmerzen und Erbrechen auf. Außerdem werden inzwischen erste Resistenzen gegen diese Medikamente beobachtet.

Da Pflanzenextrakte ein bekannt gutes Nutzen-Risiko-Verhältnis auch bei einer längeren Einnahmezeit aufweisen, wurde erfindungsgemäßer Cistus-Extrakt im Vergleich zu Zanamivir in einem in-vitro -Enzymmodell (NA-Star^{®}) gemäß Wetherall NT, Trivedi T, Zeller J, Hodges-Savola C, McKimm-Breschkin JL, Zambon M, Hayden FG. Evaluation of neuraminidase enzyme assays using different substrates to measure susceptibility of influenza virus clinical isolates to neuraminidase inhibitors: report of the neuraminidase inhibitor susceptibility network. Clin Microbiol. 2003 Feb;41(2):742-50. getestet.

Um die Effektivität des erfindungsgemäßen Extraktes an von einander unabhängigen Virenspezies zu zeigen, wurde die Neuraminidase folgender Spezies getestet:

| Virus-Spezies | IC 50 [mg/mL] Cistusextrakt 60% Polyphenole | IC 50 [µM] Zanamivir |
|---|---|---|
| Clostridium perfringens | 36,2 | >> 200 |
| Vibrio cholera | 43,1 | 200 |
| Influenza A H1N1 | 36,7 | << 0,01 |

Für den erfindungsgemäßen Extrakt konnte eine bemerkenswerte Neuraminidase-Hemmung unabhängig von der getesteten Virus-Spezies festgestellt werden. Hierdurch leiten sich die folgenden Anwendungsgebiete ab: Influenza, Cholera, Clostridien-Myositis, Enteritis necroticans.

Des weiteren wurde die sehr universelle Anwendbarkeit des erfindungsgemäßen Extraktes bei viralen Erkrankungen an Herpes-Simplex-Viren des Typ 1 (HSV-1) bewiesen:

Zur Bestimmung der viruziden Aktivität wird eine Virussuspension mit Extrakt versetzt und die Infektivität der Viren in Zellen gemessen. Als Positivkontrolle wird Ethanol verwendet.

Die Bestimmung der virustatischen Aktivität wird über den Virustiter im Überstand infizierter Humanzellen nach Beimpfung mit Extrakt ermittelt. Als anerkannte Positivkontrolle dient Aciclovir.

| Virus-Spezies | | IC 50 [µg/mL] Cistusextrakt 60% Polyphenole | IC 50 [µg/ml] Aciclovir | IC 50 [µg/ml] Ethanol |
|---|---|---|---|---|
| Herpes simplex Virus I | | | | |
| | viruzid | 3,5 | unwirksam | 80.000 |
| | virustatisch | 10,3 | 0,2 | unwirksam |

Für den erfindungsgemäßen Cistusextrakt mit 60% Polyphenolen konnte sowohl eine bemerkenswerte viruzide als auch virustatische Aktivität gemessen werden.

Überraschenderweise zeigte sich in der praktischen Anwendung auch eine Wirksamkeit bei postviralem Erschöpfungssyndrom. Die Gabe von Cistus-Tropfen (3 mal tgl. 20 Tropfen) gefertigt gemäß Bsp. 10, verzeichnete an drei männlichen Probanden im Alter von 40-42 Jahren nach Feststellung eines grippalen Infektes ein deutlich schnelleres Erholungsbild binnen 2-3 Tagen als ohne Medikation (4-7 Tage).

Für das postvirale Erschöpfungssyndrom gibt es verschiedene Namen wie Royal-Free-Erkrankung, Myalgische Enzephalomyelitis, Epidemische Neuromyasthenie, Chronische Mononukleose, Chronische Epstein-Barr-Virus-Krankheit, chronisches Erschöpfungssyndrom oder kurz postvirales Syndrom. Aktuelle Forschungen weisen darauf hin, dass die Erkrankung durch spezielle Viren hervorgerufen werden könnte, z.B. durch das Epstein-Barr-Virus, dem Verursacher des Pfeiffer'schen Drüsenfiebers. Die Symptome bilden sich meist nach Abklingen eines akuten Infektes (oft mit Fieber, Frösteln, Körperschmerzen, geschwollenen Lymphknoten und Erschöpfung) langsam aus. Nachdem die Erkrankung scheinbar ausgeklungen ist, beginnt sich der Patient wieder unwohl zu fühlen und verschiedene Beschwerden bleiben von diesem Zeitpunkt an ständig bestehen. Die Hauptsymptome sind ausgeprägte Schwäche und Schmerzen der Muskeln, Gedächtnis- und Konzentrationsschwäche, Erschöpfung und ein beständiges oder immer wiederkehrendes allgemein grippeähnliches Gefühl.

Die weitere Unterscheidung der unterschiedlichen Arten dieser postviralen Erschöpfung ist von nicht allzu großer Bedeutung. Die Krankheit wird gemäß den üblichen Prinzipien diagnostiziert und behandelt. Die Behandlung kann bei schweren Verlaufsformen jedoch viele Monate dauern, erfahrungsgemäß ist in vielen Fällen eine autonome Verbesserung der Symptome zu verzeichnen.

Weitere Anwendungsgebiete sind Influenza (auch H5N1 und H1N1) und Herpes Simplex Infektionen.

Die erfindungsgemäßen Zistrosen-Extraktzubereitungen nehmen inhibierenden Einfluss auf die NF-kB- Signaltransduktion und können bei (lokalen) allergischentzündlichen Reaktionen im HNO-Bereich eingesetzt werden. Durch die Extraktaktivität als Neuraminidase-Inhibitor sind entsprechende Extraktzubereitungen auch zur Behandlung und Prophylaxe von Rhinitis und Sinusitis als auch gegen postvirale Erschöpfungszustände verwendbar.

Als Darreichungsform für die Applikation des erfindungsgemäßen Extraktes eignen sich insbesondere Tinkturen, Sirupe, Pinselungen, Gurgellösungen, (Nasen-) Tropfen, Nasensprays sowie Inhalationslösungen oder Inhalationspulver. Ebenso sind getrocknete Formen des erfindungsgemäßen Extraktes zur üblichen Einnahme in Kapseln, Tabletten, Dragees, Pastillen, Lutsch- oder Kautabletten oder in Form rückgelöster Pulver, Granulate oder Brausezubereitungen geeignet. Als eine Darreichungsform um lokal schnell eine hohe Konzentration verfügbar zu haben, ist eine Schmelztablette bzw. sind Lyophilisate geeignet.
Figur 1 zeigt Effekte von Cistusextrakten auf die TNFalpha induzierte NF-kappaB Transkription.
Figur 2 zeigt einen HPLC-Fingerprint des Extraktes gemäß Beispiel 5.

Die zugrunde liegenden HPLC-Bedingungen sind hier aufgeführt:
Binäres Hochdruckgradientensystem, z. B. bestehend aus:

| | |
|---|---|
| 2 Pumpen: | Modell 510 bzw.515 (Fa. Waters) |
| Injektor: | Autosampler W 717 (Fa. Waters) |
| Säule: | Stahlsäule 250 x 4,6 mm Luna Phenyl-Hexyl, 5 µm (Fa. Phenomenex) |
| Säulenofen: | Jetstream Plus (Fa. VDS Optilab) |
| Detektor: | Tunable Absorbance Detektor W 2487 (Fa. Waters) |
| Integrator: | HPLC Auswerte-Software |
| Fließmittel: | A: 180 ml Acetonitril, 40 ml Essigsäure und 4,0 ml der EDTA-Lösung werden in einen 2000-ml-Messkolben gegeben und mit Wasser R fast aufgefüllt. Nach temperieren auf Raumtemperatur wird mit Wasser R bis zur Eichmarke aufgefüllt. |
| | B: 800 ml Acetonitril, 20 ml Essigsäure und 2,0 ml EDTA-Lösung werden in einen 1000-ml-Messkolben gegeben und mit Wasser R fast aufgefüllt. Nach temperieren auf Raumtemperatur wird mit Wasser R bis zur Eichmarke aufgefüllt. |
| Fluss: | 1,0 ml/min, Gradient |
| Verweilvolumen: | 2,45 ml (Modus Routine 2) |

| Flussrate [ml/min] | Zeit [min] | Eluent A [%] | Eluent B [%] | |
|---|---|---|---|---|
| 1,0 | 0 | 100 | 0 | Trennung |
| 1,0 | 10 | 100 | 0 | |
| 1,0 | 25 | 68 | 32 | |
| 1,0 | 35 | 68 | 32 | |
| 1,0 | 40 | 100 | 0 | Konditionieren |
| 1,0 | 60 | 100 | 0 | |

| | |
|---|---|
| Säulentemperatur: | 35°C |
| Injektionsvolumen: | 10 µl |
| Detektion: | Absorption im UV-Licht bei 278 nm |

Die Erfindung wird durch die folgenden Beispiele näher erläutert.

### Beispiel 1: Bewertung des Cistus-Ausgangsmaterials

Als Ausgangsdroge dienen die nachwachsenden oberirdischen Teile (Blätter, Blüten, Stängel), welche schonend getrocknet, auf eine Extraktionsgröße von etwa 10mm zerkleinert und durch eine Windsichtung von den Stängelanteilen abgereichert werden.

1000 g Ausgangsdroge werden im Perkolator mit heißem Wasser (90°C) versetzt und fünf Stunden lang extrahiert. Nach der Extraktionszeit wird abfiltriert und die Extraktion wird wiederholt. Die Extraktionszeit beträgt beim 2. Auszug drei Stunden. Beide Auszüge werden vereinigt und am Rotationsverdampfer zum Spissum eingeengt. Die Spissumextrakte der einzelnen Cistus-Spezies werden hinsichtlich ihres antioxidativen Potentials mit der DPPH-Methode untersucht.

Diese Methode basiert auf einer Redoxreaktion mit dem stabilen 2,2-Diphenyl-1-pikrylhydrazyl-Radikal (DPPH). Es kann die Fähigkeit von Extrakten als direktes Antioxidans zu wirken, bestimmt werden. Das DPPH-Radikal hat eine violette Färbung, die auf das ungepaarte Elektron am Stickstoffatom zurückzuführen ist. Die Farbe ändert sich von violett zu gelb, wenn das Radikal eine Bindung mit einem Wasserstoffatom eines Radikalfängers eingeht und das reduzierte DPPH-H (2,2-Diphenyl-1-picrylhydrazin) entsteht. Durch die photometrische Bestimmung der Verringerung der Absorption bei 517 nm kann die antioxidative Wirkung von isolierten Stoffen oder Pflanzenextrakten quantitativ bestimmt werden. Angegeben ist der 50%ige Hemmwert.

| Cistus-Spezies | IC₅₀[µg / mL] |
|---|---|
| Cistus ladanifer L. | 7,9 ± 0,7 |
| Cistus florentinus | 3,1 ± 0,2 |
| Cistus monspeliensis L. | 2,8 ± 0,2 |
| Cistus canescens | 2,0 ± 0,2 |
| Cistus creticus L. ssp. creticus | 1,3 ± 0,2 |
| Cistus creticus L. ssp. eriocephalus | 1,0 ± 0,1 |

Ergebnis: wässrige Extrakte der Spezies *Cistus creticus* L. sind den anderen Spezies bezüglich ihres antioxidativen Potentials überlegen. Besonders potent sind Extrakte der Subspecies *Cistus creticus* L. ssp. *eriocephalus.*

### Beispiel 2: Wässriger Rohextrakt

Aus 10 kg Droge Herba *Cistus creticus* L ssp. *eriocephalus,* mit einem Ausgangsgehalt von 12,3% Polyphenolen, wird durch erschöpfende Perkolation mit Wasser in 20-fachem Überschuss bei 80°C ein wässriger Extrakt hergestellt. Dieser enthält nach einer Vakuumkonzentrierung bei 50°C einen Anteil an Trockensubstanz von 60%. Die Ausbeute an nativem Extrakt beträgt 5 kg. Der Polyphenolgehalt beträgt 20,0% berechnet auf den nativen Extrakt.

### Beispiel 3: Ethanolisch-wässriger Rohextrakt

15,5 kg Ausgangsdroge Herba *Cistus creticus* L ssp. *eriocephalus* mit einem Ausgangsgehalt von 14,6% Polyphenolen, werden im Perkolator bei 40°C zweimal mit Ethanol 40% (V/V) a 1:8 erschöpfend extrahiert. Die von der Droge abgetrennten Eluate werden vereinigt, filtriert und bei 50°C schonend im Vakuum vom Lösemittel befreit. Es resultieren 6 kg wäßriger Spissumextrakt mit einem Trockensubstanzanteil von 65% (= 3,9 kg nativer Extraktanteil). Der Polyphenolgehalt beträgt 31,4% berechnet auf den nativen Extrakt.

### Beispiel 4: Extraktumlösung

5,8 kg Spissumextrakt erhalten gemäß Bsp. 3 (3,77 kg nativer Extraktanteil) werden mit demineralisiertem Wasser auf 20% Trockensubstanzanteil zurück gelöst und 60 Minuten intensiv unter Rühren homogenisiert. Anschließend wird der Ansatz für 4 h bei 10-15°C stehen gelassen. Es resultiert eine abgesetzte, unlösliche Bodenphase (5% der Gesamtmenge). Dieser Rückstand (Tannin-Fraktion) ergab einen Polyphenolgehalt von 22% bezogen auf den nativen Extrakt.

Der Überstand (Produktphase) wird von oben abgezogen und über eine CP1KS-Filterplatte klarfiltriert (Mengen-Ausbeute 95%). Der Polyphenolgehalt betrug 32,0% bezogen auf den nativen Extrakt.

### Beispiel 5: Fest - Phasen - Extraktion

Die Extraktlösung nach Bsp. 4, enthaltend 3,58 kg nativen Extrakt als ca. 20%ige Lösung, wird auf eine Säule gefüllt mit 50L Adsorberharz (Amberlite® XAD7HP) aufgebracht. Der Durchlauf wird abgetrennt und verworfen. Die Säule wird anschließend mit 3 Bettvolumen (150 L) an Wasser sauber gespült. Die Elution der adsorbierten sekundären Cistus-Inhaltsstoffe erfolgt mit 100 L Ethanol 96% (V/V). Das ethanolisch-wässrige Eluat wird aufgefangen, klarfiltriert und über eine Vakuumkonzentrierung vom Lösemittel befreit und zum Spissum beigedampft. Es resultierten 2 kg Spissum mit einem Trockensubstanzanteil von 65% (= 1,3 kg nativer Extrakt). Der Polyphenolgehalt betrug 65,0% bezogen auf den nativen Extrakt. Der Monomeren-Anteil (Theogallin, Epigallocatechin, Catechin, Epicatechin und Epigallocatechningallat) beträgt 3,6%. Der Extrakt ist durch ein spezifisches HPLC-Fingerprint gekennzeichnet (s. Fig. 2).

### Beispiel 6: Trockenextrakt

Spissum-Extrakt erhältlich nach Bsp. 5, wird einer Sprühtrocknung unterworfen. Hierzu wird der Spissum auf 40% Trockensubstanzanteil in Wasser eingestellt und einem Sprühturm mit 180°C Eingangstemperatur zugeführt. Nach der Vernebelung im Turm resultiert ein feines Pulver, das den Turm im Luftstrom bei 100°C verläßt. Die Produkttemperatur des Extraktes übersteigt dabei 55°C nicht. Es resultiert ein feines Pulver (95% < 0,315 mm) mit einem Trocknungsverlust von 4%. Der Polyphenolgehalt betrug 64,8% bezogen auf den nativen Extrakt.

### Beispiel 7: Membranfiltration

100 g Spissumextrakt erhalten gemäß Bsp. 3 (65 g nativer Extraktanteil) werden mit demineralisiertem Wasser auf 5% Trockensubstanzanteil zurück gelöst und von Schwebstoffen befreit. Es wird anschließend ein pH-Wert im Bereich von 4,5 - 5,0 eingestellt.

Diese Lösung wird mittels einer Tangentialfluß-Filtration über eine 100 kD Membran (Polyethersulfan) während 2h in zwei Fraktionen getrennt. Dabei bilden sich eine durchfiltrierte Permeat-Phase sowie eine zurückgehaltene Retentat-Phase.

Beide Phasen wurden im Vakuum zu Spissum-Extrakten beigedampft.

| Phasenbezeichnunq | Retentat | Permeat |
|---|---|---|
| Mengenverteilung bezogen auf Trockenmasse | 45% | 55% |
| Polyphenolgehalt bezogen auf nativen Extrakt | 44,4% | 15,4% |

Die Polyphenolanreicherung im Retentat verläuft spezifisch und ergibt deutlich höhere Gehalte als es die Mengenverteilung erahnen lassen würde. Gegenüber dem Einsatzextrakt mit 31,4% Polyphenolen konnte der Gehalt um 42% gesteigert werden.

### Beispiel 8: Flüssig - Flüssig - Extraktion

100 g nativer Extrakt gemäß Bsp. 3 (Retentat-Phase) wird auf 20% Trockensubstanzanteil in Wasser eingestellt. Anschließend wird bei Raumtemperatur 3x mit je 1/3 Volumen n-Butanol im Scheidetrichter intensiv vermischt. Nach einer 30 min Standzeit biidet sich ein klare Phasentrennung aus. Die n-Butanol Phase wird von der wäßrigen Phase getrennt.

Die vereinigten organischen Phasen wie auch die Wasserphase werden am Rotationsverdampfer zum Spissum eingeengt und lösungsmittelfrei gedampft.

| Phasenbezeichnunq | Organ. Phase | Wasserphase |
|---|---|---|
| Mengenverteilung bezogen auf Trockenmasse | 16% | 84% |
| Polyphenolgehalt bezogen auf nativen Extrakt | 63,7% | 27,6% |

Die Polyphenolanreicherung in der organ. Phase verläuft spezifisch und ergibt gegenüber dem Einsatzextrakt mit 31,4% Polyphenolen eine Steigerung um ca. 49%.

### Beispiel 9: Antioxidatives Potential

ORAC (Oxygen Radical Absorption capacity) ist eine international standardisierte Methode zur Bestimmung des antioxidativen Potentials. Mit dieser Methode ist eine Unterscheidung in hydrophile und lipophile Anteile der antioxidativen Wirkung möglich. Zur besseren Vergleichbarkeit wird die Gesamtkapazität auch in der Einheit des Trolox-Äquivalent (TE) angegeben. Die Durchführung ist beschrieben auf www.orac-europe.com .
Erfindungsgemäßer Cistus-Extrakt (60% Polyphenole) = 5100 µmol TE / g
Wässriger Cistus-Extrakt (25% Polyphenole) = 1900 µmol TE / g

Der erfindungsgemäß angereicherte Cistus-Extrakt mit 60% Polyphenolen besitzt ein außerordentlich hohes ORAC-Potential, daß gegenüber dem Stand der Technik um den Faktor 2,7 erhöht ist. Die Anreicherung von polyphenolhaltigen Inhaltsstoffen aus Herba Cistus L. korrelliert mit dem antioxidativen Potential.

Als Maß für oxidativen Stress in biologischen Geweben wird die Konzentration von Sauerstoffradikalen (Superoxid-Anionen) angesehen. Mittels einer Quantifizierung von Superoxid-Anionen über eine Farbstoffreduktion nach Stimulation mit Phorbol-12-myristat-13-acetat (PMA) ergeben sich folgende antioxidative Kapazitäten (Testkonzentration 200µg/ml) im Vergleich zur unbehandelten Kontrolle (0% Inaktivierung):
Erfindungsgemäßer Cistus Extrakt (60% Polyphenole): 65,0 ± 8,9%
Wässriger Cistus-Extrakt (25% Polyphenole): 20,5 ± 9,3%Vitamin C (Methoden-Standard): 96,6 ± 11,3%

Der erfindungsgemäß angereicherte Cistus-Extrakt mit 60% Polyphenolen besitzt eine außerordentlich hohe antioxidative Kapazität bei der Inaktivierung freier Sauerstoffradikale, welche gegenüber dem Stand der Technik um den Faktor 3,2 erhöht ist.

### Beispiel 10: Tropfenformulierung

Der gemäß Bsp. 5 erhaltene aufgereinigte ethanolische Extrakt wird mit Wasser auf einen Trockensubstanzanteil von 12% rückgelöst. Anschließend erfolgt die Zugabe von 20% Glycerol unter Rühren. Somit wird eine Tropfenformulierung erhalten, die geschmacklich verbessert und länger haltbar gemacht wurde.

### Beispiel 11: Anwendung als Nasentropfen

Die gemäß vorigem Bsp. 10 hergestellte Tropfenformulierung verwendete ein männlicher Proband von 38 Jahren, im Verlauf eines sehr starken akuten Heuschnupfenanfalls, nasal in Form von Tropfen aus einer Pipettenflasche. Schon nach wenigen Minuten waren die Schleimhäute auf ein normales Maß abgeschwollen.

### Beispiel 12: Anwendung als Nasenspray

Die gemäß vorigem Bsp. 10 hergestellte Tropfenformulierung verwendete ein männlicher Proband von 56 Jahren, im Verlauf eines sehr starken akuten Heuschnupfenanfalls, nasal in Form einer feinverstäubten Tröpfendosierung aus einem Verneblungsapparat. Durch diese Spray-Dosierung war schon in wenigen Minuten eine Reduzierung der Schnupfattacken erreicht worden.

## Patentansprüche

1. Verfahren zur Herstellung eines Extraktes mit mindestens 40% (m/m) Polyphenolen bezogen auf den nativen Extrakt aus *Cistus creticus L.* mit folgenden Schritten:
- Extrahieren von oberirdischen Pflanzenteilen von *Cistus creticus L.* mit einem Extraktionsmittel ausgewählt aus der Gruppe Wasser, Methanol, Ethanol, 1-Propanol, 2-Propanol und Mischungen davon, wobei der Gehalt an Alkohol nicht mehr als 50% (V/V) beträgt
- Entfernen von Extraktionsrückständen
- Zumindest teilweises Entfernen des Extraktionsmittels
- Rücklösen in einem wässrigen Lösungsmittel mit mindestens 50% (m/m) Wasser und Entfernen von unlöslichen Bestandteilen
- selektives Anreichern durch mindestens einen der folgenden Schritte
a) eine Zwei-Phasen-Extraktion, wobei
- bei einer Festphasenextraktion die Polyphenole an die Festphase binden und durch lipophilere Lösemittel eluiert werden und
- bei einer Flüssig-Flüssig-Extraktion die organische Phase die Produktphase bildet,
b) eine Membranfiltration, wobei das Retentat die Produktphase bildet.

2. Verfahren nach Anspruch 1, wobei in den Pflanzenteilen durch Mahlung und Windsichtung eine Anreicherung des Blattmaterials gegenüber Stängelanteilen erreicht wird.

3. Verfahren nach einem der vorhergehenden Ansprüche, wobei das Extraktionsmittel ausgewählt wird aus der Gruppe bestehend aus Wasser, Ethanol und Mischungen davon.

4. Verfahren nach einem der vorhergehenden Ansprüche, wobei die Extraktion bei einer Temperatur von 40 - 80°C durchgeführt wird.

5. Verfahren nach einem der vorhergehenden Ansprüche, wobei das mindestens teilweise Entfernen des Extraktionsmittels durchgeführt wird, dass der Trockensubstanzanteil > 50% (m/m) ist.

6. Verfahren nach einem der vorhergehenden Ansprüche, wobei die Zwei-Phasen-Extraktion eine Flüssig-Flüssig-Extraktion ist, insbesondere mit n-Butanol ist.

7. Verfahren nach einem der vorhergehenden Ansprüche, wobei die Zwei-Phasenextraktion eine Fest-Phasen-Extraktion ist, insbesondere an einem Adsorberharz ausgewählt aus der Gruppe bestehend aus nichtionischen hydrophoben Divinylbenzen-Copolymere, aliphatischen Esterpolymeren und Formophenolpolymeren, ist.

8. Verfahren nach einem der vorhergehenden Ansprüche, wobei die Pflanzenteile von *Cistus creticus L. ssp eriocephalus* stammen.

9. Verfahren nach einem der vorhergehenden Ansprüche, wobei der Extrakt getrocknet wird.

10. Extrakt erhältlich durch das Verfahren nach mindestens einem der Ansprüche 1 bis 9.

11. Extrakt nach Anspruch 10 mit einem nativen Droge-Extrakt-Verhältnis (DEVnativ) von 4-40:1, einem ORAC-Wert von größer 3000 µmol Trolox-Equivalent /g, einem Gesamtpolyphenolgehalt von größer 40% (m/m) sowie einem Gehalt an Monomeren von mindestens 2% (m/m).

12. Extraktzubereitung, enthaltend einen Extrakt nach Anspruch 10 oder 11 und sonstige Stoffe ausgewählt aus der Gruppe der Trägermaterialien und Antioxidantien.

13. Pharmazeutische Zubereitung oder Lebensmittel enthaltend einen Extrakt nach Anspruch 10 oder 11oder eine Extraktzubereitung nach Anspruch 12.

14. Verwendung eines Extraktes nach Anspruch 10 oder 11 oder einer Extraktzubereitung nach Anspruch 12 zur Herstellung eines Arzneimittels, Medizinproduktes oder Nahrungsergänzungsmittels zur Behandlung oder Prophylaxe bei allergisch bedingten entzündlichen Erkrankungen, Heuschnupfen, allergische Rhinitis, allergischer Konjunktivitis, bei Rhinitis, Sinusitis, Laryngitis oder Chorditis, Influenza, Cholera, Clostridien-Myositis, Enteritis necroticans, Herpes-Infektionen und/oder bei postviralen Erschöpfungszuständen.

## Claims

1. A process for preparing an extract comprising at least 40% (m/m) of polyphenols, based on the native extract, from *Cistus creticus* L., comprising the following steps:
- extracting aerial plant parts of *Cistus creticus* L. with an extractant selected from the group consisting of water, methanol, ethanol, 1-propanol, 2-propanol and mixtures thereof, the alcohol content being not more than 50% (v/v);
- removing extraction residues;
- at least partially removing the extractant;
- redissolving in an aqueous solvent having at least 50% (m/m) water, and removing insoluble components;
- selectively enriching by at least one of the following steps:
a) a two-phase extraction, wherein
- in a solid-phase extraction, the polyphenols bind to the solid phase and are eluted by more lipophilic solvents; and
- in a liquid-liquid extraction, the organic phase is the product phase;
b) a membrane filtration, the retentate being the product phase.

2. The process according to claim 1, wherein an enrichment of the leaf material over stalk fractions is achieved in the plant parts by grinding and air sweeping.

3. The process according to either of the preceding claims, wherein said extractant is selected from the group consisting of water, ethanol and mixtures thereof.

4. The process according to any of the preceding claims, wherein the extraction is performed at a temperature of from 40 to 80 °C.

5. The process according to any of the preceding claims, wherein said at least partially removing of the extractant is performed to obtain a dry matter content of > 50% (m/m).

6. The process according to any of the preceding claims, wherein said two-phase extraction is a liquid-liquid extraction, especially with n-butanol.

7. The process according to any of the preceding claims, wherein said two-phase extraction is a solid-phase extraction, especially at an adsorber resin selected from the group consisting of non-ionic hydrophobic divinylbenzene copolymers, aliphatic ester polymers and formophenol polymers.

8. The process according to any of the preceding claims, wherein said plant parts are derived from *Cistus creticus* L. ssp *eriocephalus.*

9. The process according to any of the preceding claims, wherein said extract is dried.

10. An extract obtainable by the process according to at least one of claims 1 to 9.

11. The extract according to claim 10 with a native drug-to-extract ratio (DERnative) of 4-40:1, an ORAC value of more than 3000 µmol of trolox equivalent per g, a total polyphenol content of more than 40% (m/m), and a content of monomers of at least 2% (m/m).

12. An extract formulation containing an extract according to claim 10 or 11 and other substances selected from the group consisting of support materials and antioxidants.

13. A pharmaceutical formulation or food product containing an extract according to claim 10 or 11 or an extract formulation according to claim 12.

14. Use of an extract according to claim 10 or 11 or of an extract formulation according to claim 12 for preparing a medicament, medicinal product or food supplement for the treatment or prophylaxis of allergically caused inflammatory diseases, hay fever, allergic rhinitis, allergic conjunctivitis, rhinitis, sinusitis, laryngitis or chorditis, influenza, cholera, clostridial myositis, enteritis necroticans, herpes infections and/or postviral exhaustion conditions.

## Revendications

1. Procédé de fabrication d'un extrait, avec au moins 40 % (m/m) de polyphénols par rapport à l'extrait natif de *Cistus creticus L.,* comportant les étapes suivantes :
- l'extraction de parties végétales aériennes de *Cistus creticus L.* avec un produit d'extraction choisi parmi le groupe se composant d'eau, de méthanol, d'éthanol, de propanol-1, de propanol-2 et de mélanges de ces éléments, où la teneur en alcool n'est pas de plus de 50 % (V/V)
- l'élimination de résidus d'extraction
- l'élimination au moins partielle du produit d'extraction
- la dissolution à nouveau dans un solvant aqueux avec au moins 50 % (m/m) d'eau et l'élimination de constituants insolubles
- l'enrichissement sélectif par au moins l'une des étapes suivantes
a) une extraction en deux phases, où
- au cours d'une extraction en phase stationnaire, les polyphénols se combinent à la phase stationnaire et sont élués par des solvants plus lipophiles et
- au cours d'une extraction liquide - liquide, la phase organique forme la phase de produit,
b) une filtration sur membrane où le rétentat forme la phase de produit.

2. Procédé selon la revendication 1 où, dans les parties végétales, on obtient, par broyage et par criblage à l'air, un enrichissement de la matière formée par les feuilles, par rapport aux parties formées par les tiges.

3. Procédé selon l'une ou l'autre des revendications précédentes, où le produit d'extraction est choisi parmi le groupe se composant d'eau, d'éthanol et de mélanges de ces éléments.

4. Procédé selon l'une quelconque des revendications précédentes, où l'extraction est effectuée à une température de 40°C à 80°C.

5. Procédé selon l'une quelconque des revendications précédentes, où l'élimination au moins partielle du produit d'extraction est effectuée, faisant que la proportion de substance sèche est supérieure à 50 % (m/m).

6. Procédé selon l'une quelconque des revendications précédentes, où l'extraction en deux phases est une extraction liquide - liquide, en particulier avec du n-butanol.

7. Procédé selon l'une quelconque des revendications précédentes, où l'extraction en deux phases est une extraction en phase stationnaire, en particulier sur une résine d'adsorbant choisie parmi le groupe se composant de copolymères de divinylbenzènes hydrophobes non ioniques, de polymères d'esters aliphatiques et de polymères formophénoliques.

8. Procédé selon l'une quelconque des revendications précédentes, où les parties végétales proviennent du *Cistus creticus L. ssp eriocephalus.*

9. Procédé selon l'une quelconque des revendications précédentes, où l'extrait est séché.

10. Extrait pouvant être obtenu par le procédé selon au moins l'une quelconque des revendications 1 à 9.

11. Extrait selon la revendication 10 ayant un rapport drogue / extrait natif (DER natif) de 4-40 : 1, une valeur ORAC de plus de 3000 µmol d'équivalent Trolox / g, une teneur totale en polyphénols de plus de 40 % (m/m), ainsi qu'une teneur en monomères d'au moins 2 % (m/m).

12. Préparation d'un extrait contenant un extrait selon la revendication 10 ou 11 et d'autres substances choisies parmi le groupe des matières de support et des antioxydants.

13. Préparation pharmaceutique ou produit alimentaire contenant un extrait selon la revendication 10 ou 11 ou préparation d'un extrait selon la revendication 12.

14. Utilisation d'un extrait selon la revendication 10 ou 11 ou d'une préparation d'un extrait selon la revendication 12, pour la fabrication d'un médicament, d'un produit médicinal ou d'un complément alimentaire servant au traitement ou à la prophylaxie dans le cas de maladies inflammatoires d'origine allergique, telles que les rhumes des foins, les rhinites allergiques, les conjonctivites allergiques, dans le cas de rhinites, de sinusites, de laryngites ou d'inflammations des cordes vocales, de grippes, de choléra, de myosites à clostridium, d'entérites nécrosantes, d'infections dues à des herpès et/ou dans le cas d'états d'épuisement postviraux.
